# EUROPEAN PATENT SPECIFICATION

(11) **EP 4 577 280 B1**
(45) Date of publication and mention of the grant of the patent: **06.05.2026**
(21) Application number: 23786152.1
(22) Date of filing: 05.10.2023
(51) Int. Cl.: A61M 25/00

(54) **A MICROCATHETER FOR INTERVENTIONAL CARDIOVASCULAR AND/OR NEUROVASCULAR APPLICATIONS**
MIKROKATHETER FÜR INTERVENTIONELLE KARDIOVASKULÄRE UND/ODER NEUROVASKULÄRE ANWENDUNGEN
MICROCATHÉTER POUR APPLICATIONS CARDIOVASCULAIRES ET/OU NEUROVASCULAIRES INTERVENTIONNELLES

(30) Priority: 16.11.2022 EP 22207867
(43) Date of publication of application: 02.07.2025
(73) Proprietor: IMDS R&D B.V., 9301 NZ Roden (NL)
(72) Inventor: ABBRING, Drewes Pieter, 9301 NZ Roden (NL); ELEVELD, Rolf, 9301 NZ Roden (NL); VAN DE WEG, Joachim, 9301 NZ Roden (NL); LAMERS, Edwin Adriaan Derk, 9301 NZ Roden (NL); SCHULTING, Edwin Alexander, 9301 NZ Roden (NL)
(74) Representative: V.O.
(86) International application number: PCT/NL2023/050520
(87) International publication number: WO 2024/107043

(56) References cited:
- EP-A1- 1 475 120
- US-A1- 2006 004 346
- US-A1- 2008 097 398
- US-A1- 2011 077 620

## Description

The invention relates to a catheter, being a microcatheter for interventional cardiovascular and/or neurovascular applications,
wherein the catheter comprises a tubular catheter wall, which is surrounding a lumen structure of the catheter, the catheter wall having a proximal end, a distal end and a longitudinal direction extending from the proximal end to the distal end, wherein the catheter wall has a length of at least 1000 mm from the proximal end to the distal end along the longitudinal direction;
and wherein the catheter wall comprises a proximal section, being defined as extending distally along the longitudinal direction from the proximal end to a first position at 160 mm proximal from the distal end, and at least a first distal section, being defined as extending distally along the longitudinal direction from said first position to the distal end,
and wherein at least the first distal section of the catheter wall comprises a skeleton, which is an interrupted tubular reinforcement wall obtained by microfabricating a cut pattern in a fully uninterrupted tubular reinforcement wall made of a reinforcement material, and an inner liner, which is co-axially surrounded by said skeleton relative to a center line of the catheter wall,
and wherein said skeleton provides reinforcement of the catheter wall in the longitudinal direction, as well in a circumferential direction around the longitudinal direction.

In said cardiovascular and neurovascular applications, such a catheter must be angled through the tortuous bends and curves of a vasculature passageway to arrive at the targeted anatomy. Such a catheter requires sufficient flexibility, particularly closer to its distal end, to navigate such tortuous pathways. However, other design aspects must also be considered. For example, the catheter must also be able to provide sufficient torquability (i.e., the ability to transmit torque applied at the proximal end all the way to the distal end), pushability (i.e., the ability to transmit axial push to the distal end), and structural integrity for performing intended medical functions.

US 2019/0255290 A1 discloses in Fig. 3 an example of an interventional cardiovascular catheter specifically designed for being positioned near the aortic root. Figs. 4A-4D and Fig. 5 of US 2019/0255290 A1 show some examples of skeletons that can be applied in the various longitudinal sections of the catheter of Fig. 3 of US 2019/0255290 A1, wherein such a skeleton is an interrupted tubular reinforcement wall obtained by microfabricating a cut pattern in a fully uninterrupted tubular reinforcement wall made of a reinforcement material. The catheter of Fig. 3 of US 2019/0255290 A1 has been particularly designed for providing a relatively sharp bend 54 (see Fig. 2 of US 2019/0255290 A1) to extend across the aortic root 16 and reach into the artery 24 (see Fig. 2 of US 2019/0255290 A1). Especially the proximal-intermediate section 106 of Fig. 3 of US 2019/0255290 A1), which includes the one-beam configuration of the skeleton of Fig. 4D of US 2019/0255290 A1) has been designed for providing said relatively sharp bend 54.

US 2011/077620 A1 discloses high performance guide catheters suitable for neurovascular access. They are constructed using a machined core, in which the core includes transition features from a proximal to distal-most section of the device.

US 2008/097398 A1 discloses a component for use as or incorporation within a medical instrument navigable through body vessels of a human subject. The component includes a tubular portion with an interrupted spiral defined by alternating cut and uncut sections.

EP 1 475 120 A1 discloses a tubular medical catheter having a least one region of selectively varied lamination.

US 2006/004346 A1 discloses a bend relief for supporting a cannula wherein said bend relief includes a tubular body having a region near a first end that is substantially flexible and a region near a second end that is substantially rigid. Said tubular body has a series of weakened zones disposed therealong, wherein said weakened zones provide said tubular body with regions of differential flexibility.

It is an object of the present invention to provide a solution for optimizing the flexibility of the catheter when navigating deep into the very fine cardiovascular and neurovascular tortuous pathways, while still maintaining torquability, pushability, and structural integrity of the catheter.

For that purpose the invention provides a catheter according to the appended independent claim 1. Preferable embodiments of the invention are provided by the appended dependent claims 2-14.

Accordingly, the invention provides a catheter, being a microcatheter for interventional cardiovascular and/or interventional neurovascular applications,
wherein the catheter comprises a tubular catheter wall, which is surrounding a lumen structure of the catheter, the catheter wall having a proximal end, a distal end and a longitudinal direction extending from the proximal end to the distal end, wherein the catheter wall has a length of at least 1000 mm from the proximal end to the distal end along the longitudinal direction;
and wherein the catheter wall comprises:
   - a proximal section, being defined as extending distally along the longitudinal direction from the proximal end to a first position at 160 mm proximal from the distal end,
   - a first distal section, being defined as extending distally along the longitudinal direction from said first position to the distal end,
   - a second distal section, being defined as extending distally along the longitudinal direction from a second position to the distal end, said second position being at 80 mm proximal from the distal end, and
   - a third distal section, being defined as extending distally along the longitudinal direction from a third position to the distal end, said third position being at 40 mm proximal from the distal end;
and wherein the catheter wall comprises:
   - a skeleton, which is an interrupted tubular reinforcement wall obtained by microfabricating a cut pattern in a fully uninterrupted tubular reinforcement wall made of a reinforcement material, and
   - an inner liner, which is co-axially surrounded by said skeleton relative to a center line of the catheter wall;
and wherein said skeleton provides reinforcement of the catheter wall in the longitudinal direction, as well in a circumferential direction around the longitudinal direction;
and wherein said skeleton and said inner liner are extending at least in the first distal section, the second distal section and the third distal section;
and wherein an overall skeleton cut-away ratio of a longitudinal section of the catheter wall in a longitudinal range along the longitudinal direction is defined as the percentage of:
   - the volume of all parts, within said longitudinal range, of said reinforcement material being cut-away from said fully uninterrupted tubular reinforcement wall according to said microfabricated cut pattern,
      relative to
   - the volume of all parts, within said longitudinal range, of said reinforcement material of said fully uninterrupted tubular reinforcement wall;
and wherein the overall skeleton cut-away ratio of the first distal section is at least 40%, the overall skeleton cut-away ratio of the second distal section is at least 50%, and the overall skeleton cut-away ratio of the third distal section is at least 60%.

Hence, the key features of the invention are that:
- the overall skeleton cut-away ratio of the first distal section is at least 40%,
- the overall skeleton cut-away ratio of the second distal section is at least 50%,
   and
- the overall skeleton cut-away ratio of the third distal section is at least 60%.

Thanks to these features, the microcatheter according to the invention exhibits, at least in the most distal zones of the catheter, a gradual increase of flexibility in distal direction in combination with a very high flexibility in said third distal section. Said combination of features allows for optimizing the flexibility of the catheter when navigating deep into the very fine cardiovascular and neurovascular tortuous pathways, while still maintaining torquability, pushability, and structural integrity of the catheter.

It is noted that manufacturing said very high cut-away ratios according to the invention (such as said very high cut-away ratio of 60% of the third distal section) requires inventive measures to solve several problems in the present case where the catheter concerned is a microcatheter for interventional cardiovascular and/or interventional neurovascular applications having said inner liner surrounded by said skeleton. The reason is that the inner liner for said cardiovascular and/or neurovascular applications typically is a tube made of, for example, PTFE (polytetrafluoroethylene, e.g. Teflon^{®}), PVDF (polyvinylidene fluoride, e.g. Kynar^{®}) or HDPE (high-density polyethylene), which during manufacturing of the microcatheter is axially inserted into the skeleton, and which is then expanded against the skeleton by inflation and heat. For said very high cut-away ratios the problems arise that the tube made of, for example, PTFE, PVDF or HDPE being inflated against the skeleton will bulge out of the large cut-outs in the skeleton material and/or will break under the inflation pressure. Another problem, especially for skeletons made of less strong material, is that the skeleton may severely deform and/or crack and/or break under the inflation pressure due to the fact that the skeleton is severely weakened by the large cut-outs in the skeleton material.

**In** connection therewith, the present invention inter alia is based on the new insights, as disclosed herein, that said problems can be solved by introducing, temporarily during the manufacturing of a catheter according to the invention, a heat-shrinkable outer tube over the outer side of the skeleton, so that the heat-shrinkable outer tube temporarily closes off the cut-outs in the skeleton material during said expansion of the inner liner by inflation and heat, while at the same time the heat-shrinkable outer tube temporarily reinforces the skeleton during said expansion of the inner liner by inflation and heat. After the inner liner has been successfully expanded against the skeleton and after the catheter under construction has been cooled, the temporary heat-shrinkable outer tube can be removed from the outer side of the skeleton, and, if desired, be replaced by a final outer laminate over the outer side of the skeleton.

More preferably, the overall skeleton cut-away ratio of the first distal section is at least 50%, the overall skeleton cut-away ratio of the second distal section is at least 60%, and the overall skeleton cut-away ratio of the third distal section is at least 70%.

Yet more preferably, the overall skeleton cut-away ratio of the first distal section is at least 60%, the overall skeleton cut-away ratio of the second distal section is at least 70%, and the overall skeleton cut-away ratio of the third distal section is at least 80%.

In another preferable embodiment of a catheter according to the invention, wherein the overall skeleton cut-away ratio of the first distal section is at least 40%, the overall skeleton cut-away ratio of the second distal section is at least 50%, and the overall skeleton cut-away ratio of the third distal section is at least 60%, the catheter wall further comprises:
- a fourth distal section, being defined as extending distally along the longitudinal direction from a fourth position to the distal end, said fourth position being at 20 mm proximal from the distal end, and

wherein said skeleton and said inner liner are furthermore extending in the fourth distal section,
and wherein the overall skeleton cut-away ratio of the fourth distal section is at least 70%. Thanks to these features, the microcatheter according to the invention exhibits, at least in the most distal zones of the catheter, an even more gradual increase of flexibility in distal direction in combination with an even higher flexibility in said fourth distal section, which allows for yet further optimizing the flexibility of the catheter when navigating deep into the very fine cardiovascular and neurovascular tortuous pathways, while still maintaining torquability, pushability, and structural integrity of the catheter.

More preferably, in the last-mentioned preferable embodiment the overall skeleton cut-away ratio of the first distal section is at least 50%, the overall skeleton cut-away ratio of the second distal section is at least 60%, the overall skeleton cut-away ratio of the third distal section is at least 70%, and the overall skeleton cut-away ratio of the fourth distal section is at least 80%.

Yet more preferably, in the last-mentioned preferable embodiment the overall skeleton cut-away ratio of the first distal section is at least 60%, the overall skeleton cut-away ratio of the second distal section is at least 70%, the overall skeleton cut-away ratio of the third distal section is at least 80%, and the overall skeleton cut-away ratio of the fourth distal section is at least 85%.

In yet another preferable embodiment of a catheter according to the invention, wherein the overall skeleton cut-away ratio of the first distal section is at least 40%, the overall skeleton cut-away ratio of the second distal section is at least 50%, and the overall skeleton cut-away ratio of the third distal section is at least 60%, the catheter wall further comprises:
- a fourth distal section, being defined as extending distally along the longitudinal direction from a fourth position to the distal end, said fourth position being at 20 mm proximal from the distal end, and
- a fifth distal section, being defined as extending distally along the longitudinal direction from a fifth position to the distal end, said fifth position being at 10 mm proximal from the distal end;

wherein said skeleton and said inner liner are furthermore extending in the fourth distal section and the fifth distal section;
and wherein the overall skeleton cut-away ratio of the fourth distal section is at least 70%, and the overall skeleton cut-away ratio of the fifth distal section is at least 80%. Thanks to these features, the microcatheter according to the invention exhibits, at least in the most distal zones of the catheter, a yet even more gradual increase of flexibility in distal direction in combination with a yet even higher flexibility in said fifth distal section, which allows for yet further optimizing the flexibility of the catheter when navigating deep into the very fine cardiovascular and neurovascular tortuous pathways, while still maintaining torquability, pushability, and structural integrity of the catheter.

More preferably, the overall skeleton cut-away ratio of the first distal section is at least 50%, the overall skeleton cut-away ratio of the second distal section is at least 60%, the overall skeleton cut-away ratio of the third distal section is at least 70%, the overall skeleton cut-away ratio of the fourth distal section is at least 80%, and the overall skeleton cut-away ratio of the fifth distal section is at least 85%.

Yet more preferably, the overall skeleton cut-away ratio of the first distal section is at least 60%, the overall skeleton cut-away ratio of the second distal section is at least 70%, the overall skeleton cut-away ratio of the third distal section is at least 80%, the overall skeleton cut-away ratio of the fourth distal section is at least 85%, and the overall skeleton cut-away ratio of the fifth distal section is at least 90%.

In yet another preferable embodiment of a catheter according to the invention, the inner liner is extending distally beyond the skeleton to form an atraumatic distal tip of the catheter.

In yet another preferable embodiment of a catheter according to the invention, at least the first distal section of the catheter wall further comprises an outer laminate which is co-axially surrounding said skeleton relative to the center line of the catheter wall.

More preferably, the outer laminate is extending distally beyond the skeleton to form an atraumatic distal tip of the catheter.

Yet more preferably, the inner liner and the outer laminate are extending distally beyond the skeleton to form an atraumatic distal tip of the catheter.

In yet another preferable embodiment of a catheter according to the invention, the skeleton, being said interrupted tubular reinforcement wall, comprises at least one elongated reinforcement portion, which is longitudinally extending along at least one corresponding longitudinal reinforcement path along said interrupted tubular reinforcement wall, respectively, wherein a distribution of thickness of at least one of the at least one elongated reinforcement portion progressively decreases as seen distally along the longitudinal direction of the catheter wall, wherein said thickness of the at least one elongated reinforcement portion is defined as occurring within said interrupted tubular reinforcement wall and as occurring perpendicular to the corresponding longitudinal reinforcement path. Thanks to these features, the microcatheter according to the invention exhibits a yet even more gradual increase of flexibility in distal direction, which allows for yet further optimizing the flexibility of the catheter when navigating deep into the very fine cardiovascular and neurovascular tortuous pathways, while still maintaining torquability, pushability, and structural integrity of the catheter.

In the following, the invention is further elucidated with reference to nonlimiting embodiments and with reference to the schematic figures in the appended drawing, in which the following is shown.
Fig. 1 shows, in a longitudinal side view, an example of an embodiment of a catheter according to the invention.
Fig. 2A shows a short longitudinal section of the catheter of Fig. 1, wherein the shown longitudinal section comprises a part of the skeleton of the catheter, and wherein the shown view is a longitudinal sectional view through the center line of the catheter wall of the catheter.
Fig. 2B shows a cross-section through the longitudinal section of Fig. 2A, the shown cross-section being perpendicular to the center line.
Figs. 3A-3D show, in perspective views, examples of first, second, third and fourth embodiments, respectively, of longitudinal sections of parts of a skeleton of a catheter according to the invention.

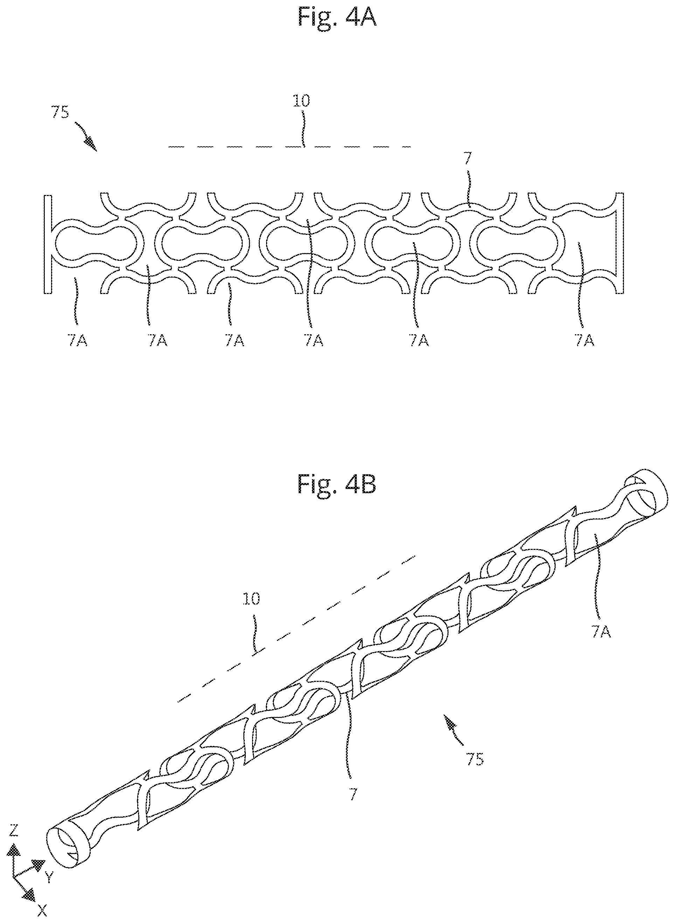

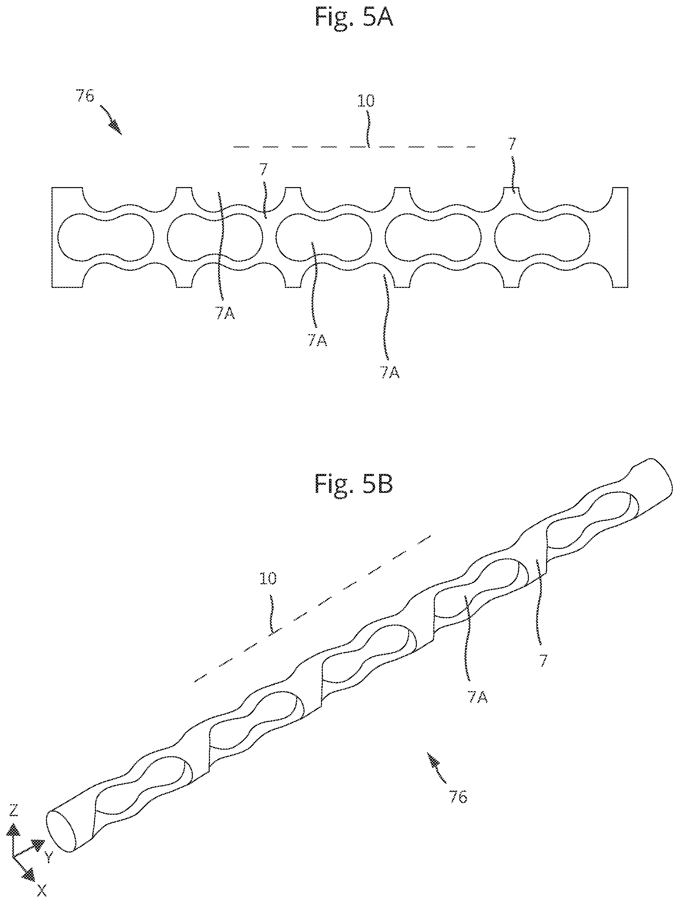

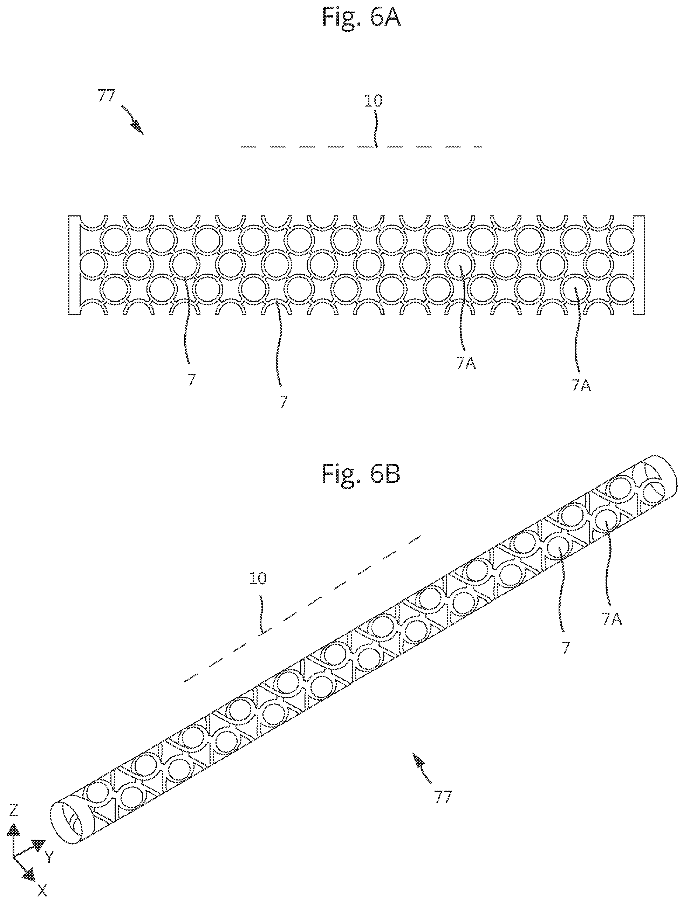

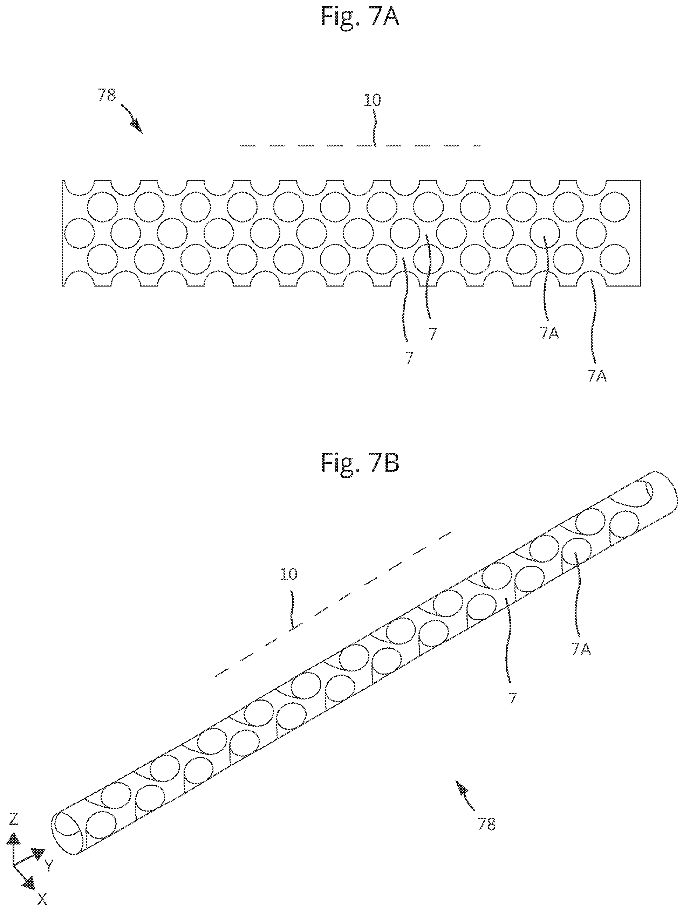

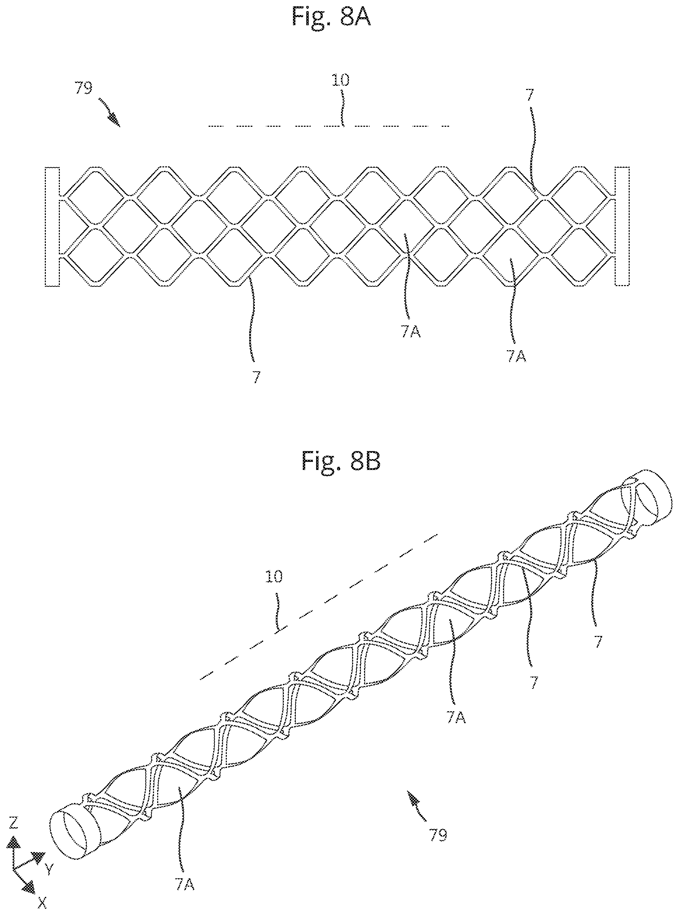

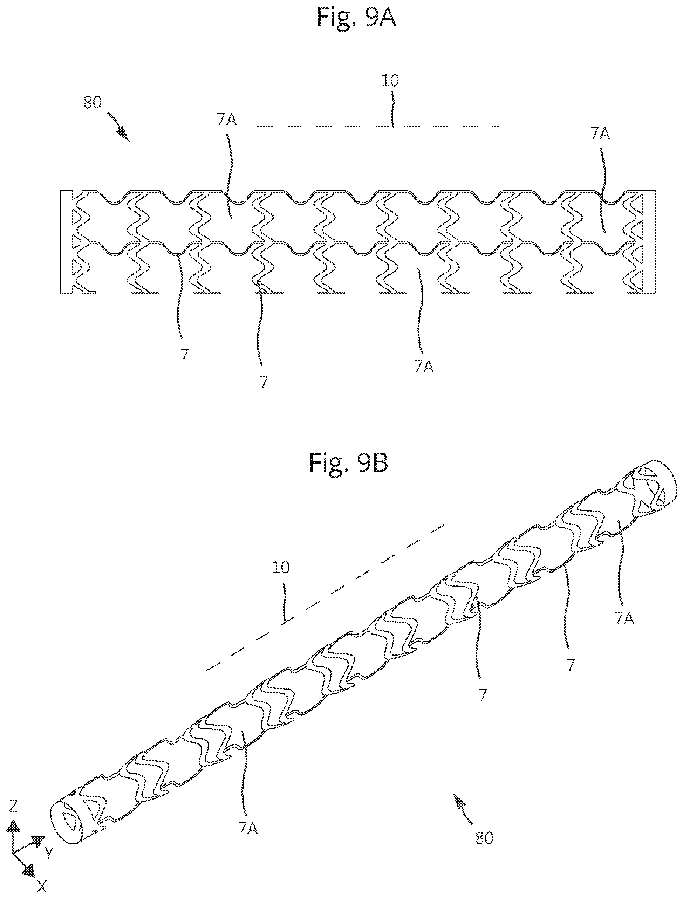

Figs. 4A, 5A, 6A, 7A, 8A and 9A show examples of fifth, sixth, seventh, eighth, ninth and tenth embodiments, respectively, of longitudinal sections of parts of a skeleton of a catheter according to the invention, wherein the shown views are top views on said longitudinal sections in imaginary "unrolled tube" states of said longitudinal sections. To explain the term "unrolled tube" it is noted that the skeleton according to the invention is a tubular wall (more particularly an interrupted tubular reinforcement wall), and that such a tubular wall imaginarily can be unrolled into a completely straight state by imaginarily unrolling it onto a fully straight plane after an imaginary straight cut, parallel to the center line, has been made over the full length of the tubular wall.
Figs. 4B, 5B, 6B, 7B, 8B and 9B show the examples of Figs. 4A, 5A, 6A, 7A, 8A and 9A, respectively, again, however this time in perspective views on their actual tubular states.
Fig. 10 shows, in a side view, an example of an eleventh embodiment of a longitudinal section of a part of a skeleton of a catheter according to the invention.

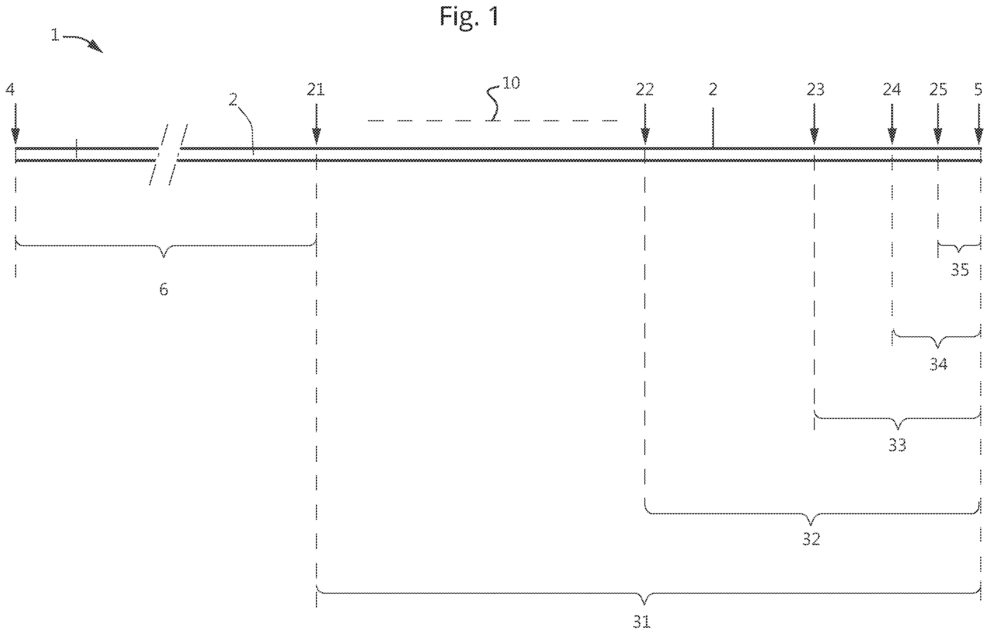

The reference numerals used in Figs. 1-10 are referring to the above-mentioned parts and aspects of the invention, as well as to related parts and aspects, in the following manner.
- 1: catheter
- 2: tubular catheter wall
- 3: lumen structure
- 4: proximal end
- 5: distal end
- 6: proximal section
- 7: skeleton
- 7A: cut pattern
- 8: inner liner
- 9: outer laminate
- 10: longitudinal direction
- 11: circumferential direction
- 12: center line
- 14, 15: elongated reinforcement portion
- 16, 17: longitudinal reinforcement path
- 21: first position
- 22: second position
- 23: third position
- 24: fourth position
- 25: fifth position
- 31: first distal section
- 32: second distal section
- 33: third distal section
- 34: fourth distal section
- 35: fifth distal section
- 71-81: first till eleventh embodiments of longitudinal sections of parts of a skeleton, respectively.

Regarding these reference signs it is noted that in the present disclosure sometimes the same reference signs have been used for like parts or aspects between different embodiments or examples occurring in Figs. 1-10.

Based on the above introductory description, including the brief description of the drawing figures, and based on the above-listed reference signs used in Figs. 1-10, the examples of Figs. 1-10 are for the greatest part readily self-explanatory. The following extra explanations are given.

Fig. 1 shows the proximal section 6, the first distal section 31, the second distal section 32, the third distal section 33, the fourth distal section 34 and the fifth distal section 35 of the tubular catheter wall 2 of the catheter 1 according to the invention. From Fig. 1 it clearly follows that the fifth distal section 35 is a distal sub-section of the fourth distal section 34, which on its turn is a distal sub-section of the third distal section 33, which on its turn is a distal sub-section of the second distal section 32, which on its turn is a distal sub-section of the first distal section 31.

As mentioned, according to the invention at least the first distal section 31 of the catheter wall comprises a skeleton and an inner liner. It is noted that also the proximal section 6 may contain a skeleton and an inner liner. As a matter of fact, the transition between the proximal section 6 and the first distal section 31 does not necessarily imply a transition in building structure between the proximal section 6 and the first distal section 31. In fact, as used herein, the definitions of the proximal section and the first, second, third, fourth and fifth distal sections, merely serve to quantify within the first distal section a specific course of the overall skeleton cut-away ratio in distal direction. In said quantification, said course of the overall skeleton cut-away ratio in distal direction follows from the occurring values of the overall skeleton cut-away ratios in said first, second and third distal sections (or in said first, second, third and fourth distal sections, as the case may be; or in said first, second, third, fourth and fifth distal sections, as the case may be).

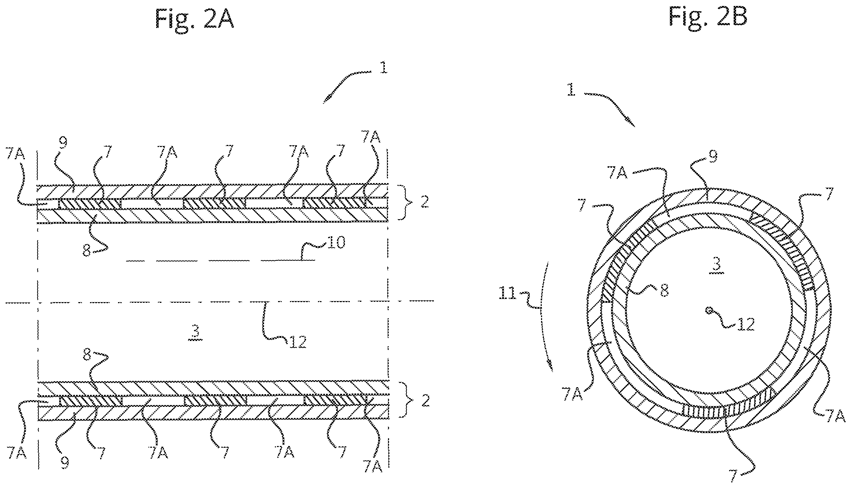

In Figs. 2A-2B it is seen that the catheter wall 2 of the catheter 1 in the shown longitudinal section of the catheter 1 of Fig. 1 has the skeleton 7 comprising the cut pattern 7A. It is further seen that the catheter wall 2 has the inner liner 8 and the outer liner 9. In the example of Figs. 2A-2B, the lumen structure 3 of the catheter 1 is a single lumen structure. Alternatively, a catheter according to the invention may have a multiple lumen structure.

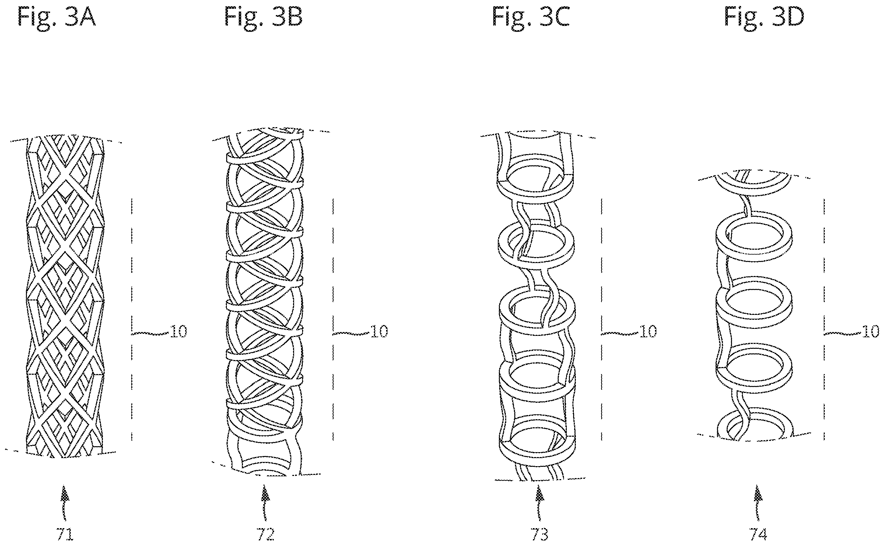

Fig. 3A shows that the skeleton 7 may at least partly be formed by the shown skeleton structure 71, which consists of eight helices, four of which being right-handed helices and four of which being left-handed helices, wherein the four right-handed helices are intersecting the four left-handed helices at multiple nodes of the skeleton structure 71.

Fig. 3B shows that the skeleton 7 may at least partly be formed by the shown skeleton structure 72, which consists of four helices, two of which being right-handed helices and two of which being left-handed helices, wherein the two right-handed helices are intersecting the two left-handed helices at multiple nodes of the skeleton structure 72.

Fig. 3C shows that the skeleton 7 may at least partly be formed by the shown skeleton structure 73, which consists of parallel rings which in axial direction are transversely inter-connected by pairs of diametrically opposite spring-like connection beams, wherein successive beam pairs in the axial direction each time are positioned with 45 degrees offset angle relative to one another in circumferential direction around the axial direction.

Fig. 3D shows that the skeleton 7 may at least partly be formed by the shown skeleton structure 74, which consists of parallel rings which in axial direction are transversely inter-connected by single spring-like connection beams, wherein successive beams in the axial direction each time are positioned with 45 degrees offset angle relative to one another in circumferential direction around the axial direction.

Based on the skeleton parts 71, 72, 73, 74 of Figs. 3A-3D, respectively, a tubular catheter wall 2 of a catheter according to the invention could, purely by way of illustrative example, have the following example skeleton. The example skeleton first has a solid hypotube starting at the proximal end 4 and having a length of 950 mm. So the solid hypotube is ending at 950 mm distal from the proximal end 4. It is noted that, as used herein, the term "solid hypotube" refers to a tubular skeleton having an overall skeleton cut-away ratio of 0%. Next, starting at the position at 950 mm distal from the proximal end 4, the skeleton has the skeleton part 71 of Fig. 3A having a length of 300 mm, so ending at 1250 mm distal from the proximal end 4. Next, starting at the position at 1250 mm distal from the proximal end 4, the skeleton has the skeleton part 72 of Fig. 3B having a length of 150 mm, so ending at 1400 mm distal from the proximal end 4. Next, starting at the position at 1400 mm distal from the proximal end 4, the skeleton has the skeleton part 73 of Fig. 3C having a length of 20 mm, so ending at 1420 mm distal from the proximal end 4. Next, starting at the position at 1420 mm distal from the proximal end 4, the skeleton has the skeleton part 74 of Fig. 3D having a length of 5 mm, so ending at 1425 mm distal from the proximal end 4. The tubular catheter wall 2 having this example skeleton could further have the above-mentioned inner liner 8 and outer laminate 9 over the full length of the tubular catheter wall 2, wherein the inner liner 8 and the outer laminate 9 are extending distally beyond the skeleton to form an atraumatic distal tip of the catheter over a length of 5 mm distally from the distal end 5 of the tubular catheter wall 2, so that the atraumatic distal tip of the catheter is ending at 1430 mm distal from the proximal end 4.

Figs. 4-9 illustrate that the skeleton 7 may at least partly be formed by many various skeleton structures, such as the shown skeleton structures 75-80 of Figs. 4-9, respectively.

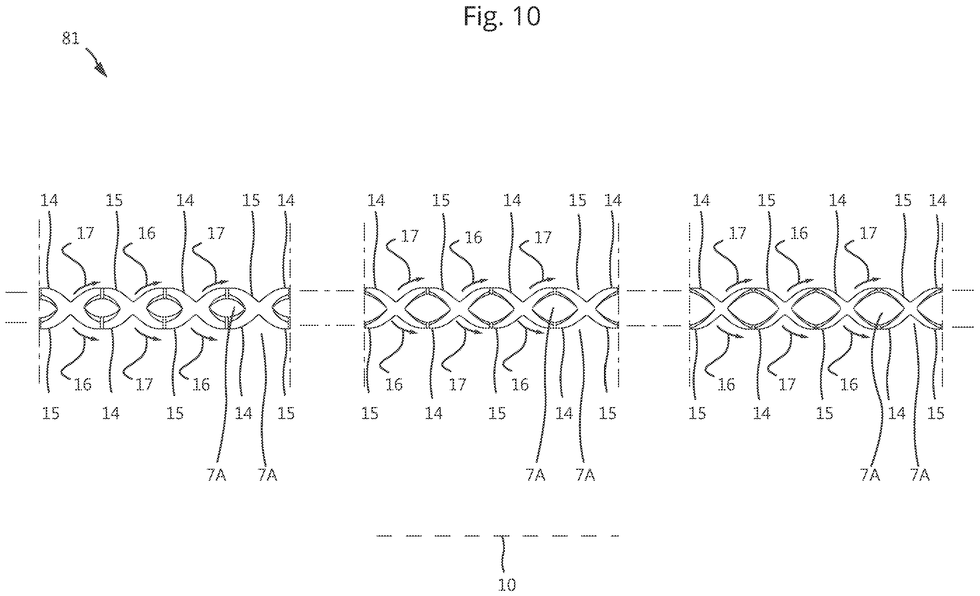

Fig. 10 shows that the skeleton 7 may at least partly be formed by the shown skeleton structure 81, which consists of two helices, i.e. a right-handed helix 14 and a left-handed helix 15, which are intersecting one another at multiple nodes of the skeleton structure 81. In Fig. 10 the direction from left to right corresponds to the distal direction along the longitudinal direction 10. The skeleton structure 81 is an example of a skeleton of a catheter according to the above-mentioned preferable embodiment of the invention, in which the skeleton comprises at least one elongated reinforcement portion (in Fig. 10 the two helices 14 and 15), which is longitudinally extending along at least one corresponding longitudinal reinforcement path (in Fig. 10 the paths 16 and 17) along said interrupted tubular reinforcement wall, respectively, wherein a distribution of thickness of at least one of the at least one elongated reinforcement portion progressively decreases as seen distally along the longitudinal direction of the catheter wall, wherein said thickness of the at least one elongated reinforcement portion is defined as occurring within said interrupted tubular reinforcement wall and as occurring perpendicular to the corresponding longitudinal reinforcement path.

The invention can be practiced with many various structures, many various materials, many various shapes and many various dimensions of the skeleton, inner liner and outer laminate of the catheter, and with many various additional features and many various accessories of the catheter, such as the many various structures, the many various materials, the many various shapes and the many various dimensions of skeletons, inner liners and outer laminates, and the many various additional features and the many various accessories that are used in known microcatheters for interventional cardiovascular and/or neurovascular applications, as long as the catheter is within the scope of the appended claims.

It is further noted that according to the invention typical dimensions of some parts and aspects of the invention, and applicable practical ranges of such dimensions are as follows. Effective length of the catheter (as measured from the distal end of a hub of the catheter) can be in the range between 130 cm and 160 cm, and can typically be 135 cm. Maximum outer diameter of the first distal section of the tubular catheter wall of the catheter can be in the range between 0.60 mm and 1.20 mm, and can typically be 0.75 mm, while noting that the word "maximum" in the term "maximum outer diameter" is referring to the maximum value as considered over the full length of the first distal section. Minimum inner diameter of the first distal section of the tubular catheter wall of the catheter can be in the range between 0.36 mm and 0.55 mm, and can typically be 0.45 mm, while noting that the word "minimum" in the term "minimum inner diameter" is referring to the minimum value as considered over the full length of the first distal section. For example, the following typical combinations of maximum outer diameter and minimum inner diameter of the first distal section are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

## Claims

1. A catheter (1), being a microcatheter for interventional cardiovascular and/or interventional neurovascular applications,
wherein the catheter comprises a tubular catheter wall (2), which is surrounding a lumen structure (3) of the catheter, the catheter wall having a proximal end (4), a distal end (5) and a longitudinal direction (10) extending from the proximal end to the distal end, wherein the catheter wall has a length of at least 1000 mm from the proximal end to the distal end along the longitudinal direction;
and wherein the catheter wall (2) comprises:
- a proximal section (6), being defined as extending distally along the longitudinal direction from the proximal end (4) to a first position (21) at 160 mm proximal from the distal end (5),
- a first distal section (31), being defined as extending distally along the longitudinal direction from said first position to the distal end,
- a second distal section (32), being defined as extending distally along the longitudinal direction from a second position (22) to the distal end (5), said second position (22) being at 80 mm proximal from the distal end (5), and
- a third distal section (33), being defined as extending distally along the longitudinal direction from a third position (23) to the distal end (5), said third position (23) being at 40 mm proximal from the distal end (5);
and wherein the catheter wall (2) comprises:
- a skeleton (7), which is an interrupted tubular reinforcement wall obtained by microfabricating a cut pattern (7A) in a fully uninterrupted tubular reinforcement wall made of a reinforcement material, and
- an inner liner (8), which is co-axially surrounded by said skeleton (7) relative to a center line (12) of the catheter wall (2);
and wherein said skeleton (7) provides reinforcement of the catheter wall (2) in the longitudinal direction (10), as well in a circumferential direction (11) around the longitudinal direction (10);
and wherein said skeleton (7) and said inner liner (8) are extending at least in the first distal section (31), the second distal section (32) and the third distal section (33);
**characterized in that**:
an overall skeleton cut-away ratio of a longitudinal section of the catheter wall (2) in a longitudinal range along the longitudinal direction (10) is defined as the percentage of:
- the volume of all parts, within said longitudinal range, of said reinforcement material being cut-away from said fully uninterrupted tubular reinforcement wall according to said microfabricated cut pattern (7A), relative to
- the volume of all parts, within said longitudinal range, of said reinforcement material of said fully uninterrupted tubular reinforcement wall;
and wherein the overall skeleton cut-away ratio of the first distal section (31) is at least 40%, the overall skeleton cut-away ratio of the second distal section (32) is at least 50%, and the overall skeleton cut-away ratio of the third distal section (33) is at least 60%.

2. The catheter (1) according to claim 1, wherein the overall skeleton cut-away ratio of the first distal section (31) is at least 50%, the overall skeleton cut-away ratio of the second distal section (32) is at least 60%, and the overall skeleton cut-away ratio of the third distal section (33) is at least 70%.

3. The catheter (1) according to claim 2, wherein the overall skeleton cut-away ratio of the first distal section (31) is at least 60%, the overall skeleton cut-away ratio of the second distal section (32) is at least 70%, and the overall skeleton cut-away ratio of the third distal section (33) is at least 80%.

4. The catheter (1) according to claim 1, wherein the catheter wall (2) further comprises:
- a fourth distal section (34), being defined as extending distally along the longitudinal direction from a fourth position (24) to the distal end (5), said fourth position (24) being at 20 mm proximal from the distal end (5), and
wherein said skeleton (7) and said inner liner (8) are furthermore extending in the fourth distal section (34),
and wherein the overall skeleton cut-away ratio of the fourth distal section (34) is at least 70%.

5. The catheter (1) according to claim 4, wherein the overall skeleton cut-away ratio of the first distal section (31) is at least 50%, the overall skeleton cut-away ratio of the second distal section (32) is at least 60%, the overall skeleton cut-away ratio of the third distal section (33) is at least 70%, and the overall skeleton cut-away ratio of the fourth distal section (34) is at least 80%.

6. The catheter (1) according to claim 5, wherein the overall skeleton cut-away ratio of the first distal section (31) is at least 60%, the overall skeleton cut-away ratio of the second distal section (32) is at least 70%, the overall skeleton cut-away ratio of the third distal section (33) is at least 80%, and the overall skeleton cut-away ratio of the fourth distal section (34) is at least 85%.

7. The catheter (1) according to claim 1, wherein the catheter wall (2) further comprises:
- a fourth distal section (34), being defined as extending distally along the longitudinal direction from a fourth position (24) to the distal end (5), said fourth position (24) being at 20 mm proximal from the distal end (5), and
- a fifth distal section (35), being defined as extending distally along the longitudinal direction (10) from a fifth position (25) to the distal end (5), said fifth position (25) being at 10 mm proximal from the distal end (5);
wherein said skeleton (7) and said inner liner (8) are furthermore extending in the fourth distal section (34) and the fifth distal section (35);
and wherein the overall skeleton cut-away ratio of the fourth distal section (34) is at least 70%, and the overall skeleton cut-away ratio of the fifth distal section (35) is at least 80%.

8. The catheter (1) according to claim 7, wherein the overall skeleton cut-away ratio of the first distal section (31) is at least 50%, the overall skeleton cut-away ratio of the second distal section (32) is at least 60%, the overall skeleton cut-away ratio of the third distal section (33) is at least 70%, the overall skeleton cut-away ratio of the fourth distal section (34) is at least 80%, and the overall skeleton cut-away ratio of the fifth distal section (35) is at least 85%.

9. The catheter (1) according to claim 8, wherein the overall skeleton cut-away ratio of the first distal section (31) is at least 60%, the overall skeleton cut-away ratio of the second distal section (32) is at least 70%, the overall skeleton cut-away ratio of the third distal section (33) is at least 80%, the overall skeleton cut-away ratio of the fourth distal section (34) is at least 85%, and the overall skeleton cut-away ratio of the fifth distal section (35) is at least 90%.

10. The catheter (1) according to any one of the preceding claims, wherein the inner liner (8) is extending distally beyond the skeleton (7) to form an atraumatic distal tip of the catheter.

11. The catheter (1) according to any one of the preceding claims, wherein at least the first distal section (31) of the catheter wall (2) further comprises an outer laminate (9) which is co-axially surrounding said skeleton (7) relative to the center line (12) of the catheter wall.

12. The catheter (1) according to claim 11, wherein the outer laminate is extending distally beyond the skeleton to form an atraumatic distal tip of the catheter.

13. The catheter (1) according to claim 11, wherein the inner liner (8) and the outer laminate (9) are extending distally beyond the skeleton (7) to form an atraumatic distal tip of the catheter.

14. The catheter (1) according to any one of the preceding claims, wherein the skeleton (81), being said interrupted tubular reinforcement wall, comprises at least one elongated reinforcement portion (14, 15), which is longitudinally extending along at least one corresponding longitudinal reinforcement path (16, 17) along said interrupted tubular reinforcement wall, respectively, wherein a distribution of thickness of at least one of the at least one elongated reinforcement portion (14, 15) progressively decreases as seen distally along the longitudinal direction (10) of the catheter wall (2), wherein said thickness of the at least one elongated reinforcement portion (14, 15) is defined as occurring within said interrupted tubular reinforcement wall and as occurring perpendicular to the corresponding longitudinal reinforcement path (16, 17).

## Patentansprüche

1. Katheter (1), der ein Mikrokatheter für interventionelle kardiovaskuläre und/oder neurovaskuläre Anwendungen ist,
wobei der Katheter eine röhrenförmige Katheterwand (2) umfasst, die eine Lumenstruktur (3) des Katheters umgibt, wobei die Katheterwand ein proximales Ende (4), ein distales Ende (5) und eine Längsrichtung (10) aufweist, die sich vom proximalen Ende zum distalen Ende erstreckt, wobei die Katheterwand entlang der Längsrichtung eine Länge von mindestens 1000 mm vom proximalen Ende zum distalen Ende aufweist;
und wobei die Katheterwand (2) umfasst:
- einen proximalen Abschnitt (6), der als sich distal entlang der Längsrichtung vom proximalen Ende (4) zu einer ersten Position (21) bei 160 mm proximal vom distalen Ende (5) erstreckend definiert ist,
- einen ersten distalen Abschnitt (31), der als sich distal entlang der Längsrichtung von der ersten Position zum distalen Ende erstreckend definiert ist,
- einen zweiten distalen Abschnitt (32), der als sich distal entlang der Längsrichtung von einer zweiten Position (22) zum distalen Ende (5) erstreckend definiert ist, wobei die zweite Position (22) 80 mm proximal vom distalen Ende (5) entfernt ist, und
- einen dritten distalen Abschnitt (33), der als sich distal entlang der Längsrichtung von einer dritten Position (23) zum distalen Ende (5) erstreckend definiert ist, wobei sich die dritte Position (23) 40 mm proximal vom distalen Ende (5) befindet;
und wobei die Katheterwand (2) umfasst:
- ein Gerüst (7), das eine unterbrochene röhrenförmige Verstärkungswand ist, die durch Mikrofabrikation eines Schnittmusters (7A) in einer vollständig ununterbrochenen röhrenförmigen Verstärkungswand aus einem Verstärkungsmaterial erhalten wird, und
- eine Innenauskleidung (8), die koaxial von dem Gerüst (7) relativ zu einer Mittellinie (12) der Katheterwand umgeben ist (2);
und wobei das Gerüst (7) eine Verstärkung der Katheterwand (2) in Längsrichtung (10) sowie in Umfangsrichtung (11) um die Längsrichtung (10) herum bereitstellt;
und wobei sich das Gerüst (7) und die Innenauskleidung (8) mindestens im ersten distalen Abschnitt (31), im zweiten distalen Abschnitt (32) und im dritten distalen Abschnitt (33) erstrecken;
**dadurch gekennzeichnet, dass**:
ein Gesamtgerüst-Ausschnittverhältnis eines Längsschnitts der Katheterwand (2) in einem Längsbereich entlang der Längsrichtung (10) definiert ist als der Prozentsatz von:
- dem Volumen aller Teile innerhalb des Längsbereichs des Verstärkungsmaterials, die gemäß dem mikrogefertigten Schnittmuster (7A) aus der vollständig ununterbrochenen röhrenförmigen Verstärkungswand ausgeschnitten sind,
im Verhältnis
- zum Volumen aller Teile innerhalb des Längsbereichs des Verstärkungsmaterials der vollständig ununterbrochenen röhrenförmigen Verstärkungswand;
und wobei das Gesamtgerüst-Ausschnittverhältnis des ersten distalen Abschnitts (31) mindestens 40 % beträgt, das Gesamtgerüst-Ausschnittverhältnis des zweiten distalen Abschnitts (32) mindestens 50 % beträgt und das Gesamtgerüst-Ausschnittverhältnis des dritten distalen Abschnitts (33) mindestens 60 % beträgt.

2. Katheter (1) nach Anspruch 1, wobei das Gesamtgerüst-Ausschnittverhältnis des ersten distalen Abschnitts (31) mindestens 50 % beträgt, das Gesamtgerüst-Ausschnittverhältnis des zweiten distalen Abschnitts (32) mindestens 60 % beträgt und das Gesamtgerüst-Ausschnittverhältnis des dritten distalen Abschnitts (33) mindestens 70 % beträgt.

3. Katheter (1) nach Anspruch 2, wobei das Gesamtgerüst-Ausschnittverhältnis des ersten distalen Abschnitts (31) mindestens 60 % beträgt, das Gesamtgerüst-Ausschnittverhältnis des zweiten distalen Abschnitts (32) mindestens 70 % beträgt und das Gesamtgerüst-Ausschnittverhältnis des dritten distalen Abschnitts (33) mindestens 80 % beträgt.

4. Katheter (1) nach Anspruch 1, wobei die Katheterwand (2) ferner umfasst:
- einen vierten distalen Abschnitt (34), der als sich distal entlang der Längsrichtung von einer vierten Position (24) zum distalen Ende (5) erstreckend definiert ist, wobei die vierte Position (24) 20 mm proximal vom distalen Ende (5) entfernt ist, und wobei sich das Gerüst (7) und die Innenauskleidung (8) ferner
in den vierten distalen Abschnitt (34) erstrecken,
und wobei das Gesamtgerüst-Ausschnittverhältnis des vierten distalen Abschnitts (34) mindestens 70 % beträgt.

5. Katheter (1) nach Anspruch 4, wobei das Gesamtgerüst-Ausschnittverhältnis des ersten distalen Abschnitts (31) mindestens 50 % beträgt, das Gesamtgerüst-Ausschnittverhältnis des zweiten distalen Abschnitts (32) mindestens 60 % beträgt, das Gesamtgerüst-Ausschnittverhältnis des dritten distalen Abschnitts (33) mindestens 70 % beträgt und das Gesamtgerüst-Ausschnittverhältnis des vierten distalen Abschnitts (34) mindestens 80 % beträgt.

6. Katheter (1) nach Anspruch 5, wobei das Gesamtgerüst-Ausschnittverhältnis des ersten distalen Abschnitts (31) mindestens 60 % beträgt, das Gesamtgerüst-Ausschnittverhältnis des zweiten distalen Abschnitts (32) mindestens 70 % beträgt, das Gesamtgerüst-Ausschnittverhältnis des dritten distalen Abschnitts (33) mindestens 80 % beträgt und das Gesamtgerüst-Ausschnittverhältnis des vierten distalen Abschnitts (34) mindestens 85 % beträgt.

7. Katheter (1) nach Anspruch 1, wobei die Katheterwand (2) ferner umfasst:
- einen vierten distalen Abschnitt (34), der als sich distal entlang der Längsrichtung von einer vierten Position (24) zum distalen Ende (5) erstreckend definiert ist, wobei die vierte Position (24) 20 mm proximal vom distalen Ende (5) entfernt ist, und
- einen fünften distalen Abschnitt (35), der als sich distal entlang der Längsrichtung (10) von einer fünften Position (25) zum distalen Ende (5) erstreckend definiert ist, wobei die fünfte Position (25) 10 mm proximal vom distalen Ende (5) entfernt ist;
wobei sich das Gerüst (7) und die Innenauskleidung (8) ferner in den vierten distalen Abschnitt (34) und den fünften distalen Abschnitt (35) erstrecken;
und wobei das Gesamtgerüst-Ausschnittverhältnis im vierten distalen Abschnitt (34) mindestens 70 % beträgt und das Gesamtgerüst-Ausschnittverhältnis im fünften distalen Abschnitt (35) mindestens 80 % beträgt.

8. Katheter (1) nach Anspruch 7, wobei das Gesamtgerüst-Ausschnittverhältnis des ersten distalen Abschnitts (31) mindestens 50 % beträgt, das Gesamtgerüst-Ausschnittverhältnis des zweiten distalen Abschnitts (32) mindestens 60 % beträgt, das Gesamtgerüst-Ausschnittverhältnis des dritten distalen Abschnitts (33) mindestens 70 % beträgt, das Gesamtgerüst-Ausschnittverhältnis des vierten distalen Abschnitts (34) mindestens 80 % beträgt und das Gesamtgerüst-Ausschnittverhältnis des fünften distalen Abschnitts (35) mindestens 85 % beträgt.

9. Katheter (1) nach Anspruch 8, wobei das Gesamtgerüst-Ausschnittverhältnis des ersten distalen Abschnitts (31) mindestens 60 % beträgt, das Gesamtgerüst-Ausschnittverhältnis des zweiten distalen Abschnitts (32) mindestens 70 % beträgt, das Gesamtgerüst-Ausschnittverhältnis des dritten distalen Abschnitts (33) mindestens 80 % beträgt, das Gesamtgerüst-Ausschnittverhältnis des vierten distalen Abschnitts (34) mindestens 85 % beträgt und das Gesamtgerüst-Ausschnittverhältnis des fünften distalen Abschnitts (35) mindestens 90 % beträgt.

10. Katheter (1) nach einem der vorstehenden Ansprüche, wobei sich die Innenauskleidung (8) distal über das Gerüst (7) hinaus erstreckt, um eine atraumatische distale Spitze des Katheters zu bilden.

11. Katheter (1) nach einem der vorstehenden Ansprüche, wobei zumindest der erste distale Abschnitt (31) der Katheterwand (2) ferner ein Außenlaminat (9) umfasst, das das Gerüst (7) relativ zur Mittellinie (12) der Katheterwand koaxial umgibt.

12. Katheter (1) gemäß Anspruch 11, wobei sich das Außenlaminat distal über das Gerüst hinaus erstreckt, um eine atraumatische distale Spitze des Katheters zu bilden.

13. Katheter (1) nach Anspruch 11, wobei sich die Innenauskleidung (8) und das Außenlaminat (9) distal über das Gerüst (7) hinaus erstrecken, um eine atraumatische distale Spitze des Katheters zu bilden.

14. Katheter (1) nach einem der vorstehenden Ansprüche, wobei das Gerüst (81), das die unterbrochene röhrenförmige Verstärkungswand ist, mindestens einen länglichen Verstärkungsabschnitt (14, 15) umfasst, der sich in Längsrichtung entlang mindestens eines entsprechenden Längsverstärkungswegs (16, 17) entlang der unterbrochenen röhrenförmigen Verstärkungswand erstreckt, wobei eine Verteilung der Dicke mindestens eines der mindestens einen länglichen Verstärkungsabschnitte (14, 15) in distaler Richtung entlang der Längsrichtung (10) der Katheterwand (2) zunehmend abnimmt, wobei die Dicke des mindestens einen länglichen Verstärkungsabschnitts (14, 15) als innerhalb der unterbrochenen röhrenförmigen Verstärkungswand und senkrecht zum entsprechenden Längsverstärkungsweg (16, 17) auftretend definiert ist.

## Revendications

1. Cathéter (1), qui est un microcathéter destiné à des applications cardiovasculaires et/ou neurovasculaires interventionnelles,
dans lequel le cathéter comprend une paroi de cathéter tubulaire (2), qui entoure une structure de lumière (3) du cathéter, la paroi de cathéter ayant une extrémité proximale (4), une extrémité distale (5) et une direction longitudinale (10) s'étendant de l'extrémité proximale à l'extrémité distale, dans lequel la paroi de cathéter a une longueur d'au moins 1000 mm de l'extrémité proximale à l'extrémité distale le long de la direction longitudinale ;
et dans lequel la paroi de cathéter (2) comprend :
- une section proximale (6), définie comme s'étendant distalement le long de la direction longitudinale depuis l'extrémité proximale (4) jusqu'à une première position (21) située à 160 mm en proximal de l'extrémité distale (5),
- une première section distale (31), définie comme s'étendant distalement le long de la direction longitudinale depuis ladite première position jusqu'à l'extrémité distale,
- une deuxième section distale (32), définie comme s'étendant distalement le long de la direction longitudinale depuis une deuxième position (22) jusqu'à l'extrémité distale (5), ladite deuxième position (22) étant située à 80 mm en proximal de l'extrémité distale (5), et
- une troisième section distale (33), définie comme s'étendant distalement le long de la direction longitudinale depuis une troisième position (23) jusqu'à l'extrémité distale (5), ladite troisième position (23) étant située à 40 mm en proximal de l'extrémité distale (5) ;
et dans lequel la paroi de cathéter (2) comprend :
- un squelette (7), qui est une paroi de renfort tubulaire interrompue obtenue par microfabrication d'un motif de découpe (7A) dans une paroi de renfort tubulaire entièrement ininterrompue faite d'un matériau de renfort, et
- une doublure intérieure (8), qui est entourée coaxialement par ledit squelette (7) par rapport à une ligne centrale (12) de la paroi de cathéter (2) ;
et dans lequel ledit squelette (7) assure le renforcement de la paroi de cathéter (2) dans la direction longitudinale (10), ainsi que dans une direction circonférentielle (11) autour de la direction longitudinale (10) ;
et dans lequel ledit squelette (7) et ladite doublure intérieure (8) s'étendent au moins dans la première section distale (31), la deuxième section distale (32) et la troisième section distale (33) ;
**caractérisé en ce que** :
un rapport global de découpe de squelette d'une section longitudinale de la paroi de cathéter (2) dans une plage longitudinale le long de la direction longitudinale (10) est défini comme étant le pourcentage :
- du volume de toutes les parties, au sein de ladite plage longitudinale, dudit matériau de renfort découpé de ladite paroi de renfort tubulaire entièrement ininterrompue selon ledit motif de découpe microfabriqué (7A),
par rapport
- au volume de toutes les parties, dans ladite plage longitudinale, dudit matériau de renfort de ladite paroi de renfort tubulaire entièrement ininterrompue ;
et dans lequel le rapport global de découpe de squelette de la première section distale (31) est d'au moins 40 %, le rapport global de découpe de squelette de la deuxième section distale (32) est d'au moins 50 %, et le rapport global de découpe de squelette de la troisième section distale (33) est d'au moins 60 %.

2. Cathéter (1) selon la revendication 1, dans lequel le rapport global de découpe de squelette de la première section distale (31) est d'au moins 50 %, le rapport global de découpe de squelette de la deuxième section distale (32) est d'au moins 60 % et le rapport global de découpe de squelette de la troisième section distale (33) est d'au moins 70 %.

3. Cathéter (1) selon la revendication 2, dans lequel le rapport global de découpe de squelette de la première section distale (31) est d'au moins 60 %, le rapport global de découpe de squelette de la deuxième section distale (32) est d'au moins 70 % et le rapport global de découpe de squelette de la troisième section distale (33) est d'au moins 80 %.

4. Cathéter (1) selon la revendication 1, dans lequel la paroi de cathéter (2) comprend en outre :
- une quatrième section distale (34), définie comme s'étendant distalement le long de la direction longitudinale depuis une quatrième position (24) jusqu'à l'extrémité distale (5), ladite quatrième position (24) étant située à 20 mm en proximal de l'extrémité distale (5), et
dans lequel ledit squelette (7) et ladite doublure intérieure (8) s'étendent en outre dans la quatrième section distale (34),
et dans lequel le rapport global de découpe de squelette de la quatrième section distale (34) est d'au moins 70 %.

5. Cathéter (1) selon la revendication 4, dans lequel le rapport global de découpe de squelette de la première section distale (31) est d'au moins 50 %, le rapport global de découpe de squelette de la deuxième section distale (32) est d'au moins 60 %, le rapport global de découpe de squelette de la troisième section distale (33) est d'au moins 70 %, et le rapport global de découpe de squelette de la quatrième section distale (34) est d'au moins 80 %.

6. Cathéter (1) selon la revendication 5, dans lequel le rapport global de découpe de squelette de la première section distale (31) est d'au moins 60 %, le rapport global de découpe de squelette de la deuxième section distale (32) est d'au moins 70 %, le rapport global de découpe de squelette de la troisième section distale (33) est d'au moins 80 %, et le rapport global de découpe de squelette de la quatrième section distale (34) est d'au moins 85 %.

7. Cathéter (1) selon la revendication 1, dans lequel la paroi de cathéter (2) comprend en outre :
- une quatrième section distale (34), définie comme s'étendant distalement le long de la direction longitudinale depuis une quatrième position (24) jusqu'à l'extrémité distale (5), ladite quatrième position (24) étant située à 20 mm en proximal de l'extrémité distale (5), et
- une cinquième section distale (35), définie comme s'étendant distalement le long de la direction longitudinale (10) depuis une cinquième position (25) jusqu'à l'extrémité distale (5), ladite cinquième position (25) étant située à 10 mm en proximal de l'extrémité distale (5) ;
dans lequel ledit squelette (7) et ladite doublure intérieure (8) s'étendent en outre dans la quatrième section distale (34) et la cinquième section distale (35) ;
et dans lequel le rapport global de découpe de squelette de la quatrième section distale (34) est d'au moins 70 %, et le rapport global de découpe de squelette de la cinquième section distale (35) est d'au moins 80 %.

8. Cathéter (1) selon la revendication 7, dans lequel le rapport global de découpe de squelette de la première section distale (31) est d'au moins 50 %, le rapport global de découpe de squelette de la deuxième section distale (32) est d'au moins 60 %, le rapport global de découpe de squelette de la troisième section distale (33) est d'au moins 70 %, le rapport global de découpe de squelette de la quatrième section distale (34) est d'au moins 80 %, et le rapport global de découpe de squelette de la cinquième section distale (35) est d'au moins 85 %.

9. Cathéter (1) selon la revendication 8, dans lequel le rapport global de découpe de squelette de la première section distale (31) est d'au moins 60 %, le rapport global de découpe de squelette de la deuxième section distale (32) est d'au moins 70 %, le rapport global de découpe de squelette de la troisième section distale (33) est d'au moins 80 %, le rapport global de découpe de squelette de la quatrième section distale (34) est d'au moins 85 %, et le rapport global de découpe de squelette de la cinquième section distale (35) est d'au moins 90 %.

10. Cathéter (1) selon l'une quelconque des revendications précédentes, dans lequel la doublure intérieure (8) s'étend distalement au-delà du squelette (7) afin de former une pointe distale atraumatique du cathéter.

11. Cathéter (1) selon l'une quelconque des revendications précédentes, dans lequel au moins la première section distale (31) de la paroi de cathéter (2) comprend en outre un stratifié extérieur (9) qui entoure coaxialement ledit squelette (7) par rapport à la ligne centrale (12) de la paroi de cathéter.

12. Cathéter (1) selon la revendication 11, dans lequel le stratifié extérieur s'étend distalement au-delà du squelette afin de former une pointe distale atraumatique du cathéter.

13. Cathéter (1) selon la revendication 11, dans lequel la doublure intérieure (8) et le stratifié extérieur (9) s'étendent distalement au-delà du squelette (7) afin de former une pointe distale atraumatique du cathéter.

14. Cathéter (1) selon l'une quelconque des revendications précédentes, dans lequel le squelette (81), qui est ladite paroi de renfort tubulaire interrompue, comprend au moins une partie de renfort allongée (14, 15), qui s'étend longitudinalement le long d'au moins un chemin de renfort longitudinal correspondant (16, 17) le long de ladite paroi de renfort tubulaire interrompue, respectivement, dans lequel une distribution d'épaisseur d'au moins une de l'au moins une partie de renfort allongée (14, 15) diminue progressivement telle qu'observée distalement le long de la direction longitudinale (10) de la paroi de cathéter (2), dans laquelle ladite épaisseur de l'au moins une partie de renfort allongée (14, 15) est définie comme se produisant à l'intérieur de ladite paroi de renfort tubulaire interrompue et comme se produisant perpendiculairement au chemin de renfort longitudinal correspondant (16, 17).
